# EUROPEAN PATENT APPLICATION

(11) **EP 4 732 854 A2**
(43) Date of publication of application: **29.04.2026**
(21) Application number: 25211470.7
(22) Date of filing: 27.10.2025
(51) Int. Cl.: A61L 9/00, A01N 25/12, A41D 31/30, B01D 39/20

(54) **BACTERIA AND VIRUS INHIBITING ARRANGEMENT**

(30) Priority: 28.10.2024 SE 2451064
(71) Applicant: Xodor AB, 120 08 Stockholm (SE)
(72) Inventor: THILANDER, Claes, 76197 Norrtälje (SE)
(74) Representative: Brann AB

(57) **Abstract**

The present disclosure relates to a bacteria and virus inhibiting arrangement (100) for through-flowing fluids, the arrangement comprising: a granular substance (110) comprising crushed and calcinated mineral, wherein a pH of the granular substance is above 10, a permeable enclosure (120) configured to hold the granular substance and to partially enclose fluid flowing through the granular substance to expose external surfaces of the granular substance to the fluid.

## Description

The present invention relates to a bacteria and virus inhibiting arrangement.

### BACKGROUND

Fluid is frequently transported to and from closed spaces, such as rooms of a building, an interior of vehicles or even lungs of a person.

The fluid is typically transported to or from the spaces via drains and vents.

In one example, a floor drain is used to transport fluid in the form of water out of a room. The floor drain is typically provided with a water lock to prevent odors emanating from the sewer that the floor drain is coupled to. A drawback is that the water lock loses its functionality when the floor drain is not used for some time, and sewer gases, bacteria and virus may flow into the closed space.

In one further example, an air vent is used to transport fluid in the form of air into a room. The air vent or any of the coupled air ducts is typically provided with an air filter to prevent or reduce dust or particles entering the closed space. A drawback is that bad smelling gas may be formed in the air ducts, e.g., due to bacteria, and , and the bad smelling gas together with bacteria and virus may flow into the closed space.

Conventional solutions have suggested adding arrangements using agents reducing odors, e.g., using active coal. However, this provides limited flow of fluid, e.g., in a floor drain. It also only addresses unwanted effects of bad odor, not the root cause.

Thus, there is a need for an improved arrangement.

### OBJECTS OF THE INVENTION

An objective of embodiments of the present invention is to provide a solution which mitigates or solves the drawbacks described above.

### SUMMARY OF THE INVENTION

The above and further objectives are achieved by the subject matter described herein. Further advantageous implementation forms of the invention are described herein. The invention is set out in the appended claims. The scope of the invention is defined by the claims, which are incorporated into this section by reference.

According to a first aspect of the invention, the above mentioned objective is achieved by a fungi, bacteria and virus inhibiting arrangement, the arrangement comprising: a granular substance comprising crushed and calcinated mineral, wherein a pH of the granular substance is above 10, a permeable enclosure configured to hold the granular substance and to partially enclose fluid flowing through the granular substance to expose external surfaces of the granular substance to the fluid.

In one embodiment according to the first aspect, the granular substance comprises Calcium silicate.

In one embodiment according to the first aspect, fractions of the granular substance are selected in proportion to a target flow of the fluid through the arrangement.

In one embodiment according to the first aspect, fractions of the granular substance are selected within a range of 0.001 millimeters to 10 millimeters.

In one embodiment according to the first aspect, the shape of the permeable enclosure is adapted to an outline of a floor drain to force fluid emanating from the floor drain to pass through the arrangement and be exposed to the granular substance.

In one embodiment according to the first aspect, the shape of the permeable enclosure is adapted to an interior of the floor drain.

In one embodiment according to the first aspect, the shape of the permeable enclosure is adapted to an outline of a cover of the floor drain.

In one embodiment according to the first aspect, the shape of the permeable enclosure is adapted to a vent, to force fluid emanating from the vent to pass through the arrangement and be exposed to the granular substance.

In one embodiment according to the first aspect, the shape of the permeable enclosure is adapted to a face mask, to force fluid inhaled by a user of the face mask to pass through the arrangement and be exposed to the granular substance.

In one embodiment according to the first aspect, the shape of the permeable enclosure is adapted to a filter to force fluid received from an inlet to pass through the arrangement and be exposed to the granular substance.

In one embodiment according to the first aspect, the permeable enclosure is adapted to an air-filter of a vehicle.

In one embodiment according to the first aspect, the permeable enclosure comprises a first permeable part, a second permeable part and a third non-permeable part, wherein the third non-permeable part is joined to the first permeable part and to the second permeable part to hold the granular substance.

Reference will be made to the appended sheets of drawings that will first be described briefly. It should be appreciated that, like reference numerals are used to identify like elements illustrated in one or more of the drawings.

### BRIEF DESCRIPTION OF THE DRAWINGS

**Fig. 1** shows a bacteria and virus inhibiting arrangement according to one or more embodiments of the present disclosure.
**Fig. 2** shows details of the arrangement according to one or more embodiments of the present disclosure.
**Fig. 3** illustrates an example where the shape of the permeable enclosure is adapted to an outline of a floor drain according to the present disclosure.
**Fig. 4AB** illustrates an embodiment where the shape of the permeable enclosure is adapted to an interior of a floor drain according to the present disclosure.
**Fig. 5** illustrates a side view of an embodiment where the shape of the permeable enclosure is adapted to a vent or a filter according to the present disclosure.
**Fig. 6** illustrates a perspective view of an embodiment where the shape of the permeable enclosure is adapted to an air filter of a vehicle according to the present disclosure.
**Fig. 7** illustrates a perspective view of an embodiment where the shape of the permeable enclosure is adapted to a face mask according to the present disclosure.
**Fig. 8** **and** **Fig. 9** illustrate comparisons between not using and using the arrangement according to the present disclosure.

A more complete understanding of embodiments of the invention will be afforded to those skilled in the art, as well as a realization of additional advantages thereof, by a consideration of the following detailed description of one or more embodiments. It should be appreciated that like reference numerals are used to identify like elements illustrated in one or more of the figures.

### DETAILED DESCRIPTION

Generally, all terms used herein are to be interpreted according to their ordinary meaning in the relevant technical field, unless a different meaning is clearly given and/or is implied from the context in which it is used. All references to a/an/the element, apparatus, component, means, step, etc. are to be interpreted openly as referring to at least one instance of the element, apparatus, component, means, step, etc., unless explicitly stated otherwise. The steps of any methods disclosed herein do not have to be performed in the exact order disclosed, unless a step is explicitly described as following or preceding another step and/or where it is implicit that a step must follow or precede another step. Any feature of any of the embodiments disclosed herein may be applied to any other embodiment, wherever appropriate. Likewise, any advantage of any of the embodiments may apply to any other embodiments, and vice versa. Other objectives, features and advantages of the enclosed embodiments will be apparent from the following description.

An "or" in this description and the corresponding claims is to be understood as a mathematical OR which covers "and" and "or", and is not to be understand as an XOR (exclusive OR). The indefinite article "a" in this disclosure and claims is not limited to "one" and can also be understood as "one or more", i.e., plural.

In the present disclosure, the term "granular substance" defines a substance that is composed of a lot of granules or particles having a maximum size and/or a minimum size.

In the present disclosure, the term "fraction" or "aggregate" defines a maximum size and/or a minimum size of particles or granules of the granular substance. The fraction of granular substance particles may be generated by crushing material/mineral/ore to a particular size and sifting the crushed material/mineral/ore to separate a particular fraction, the fraction comprising the maximum size and/or a minimum size of particles or granules.

In one example, particles having a minimum circumferential size of 0.5 millimetres (mm) and a maximum circumferential size of 0.6 mm are selected to a fraction of granular substance.

Additionally, or alternatively, the fraction of granular substance particles may further be generated by adding a binder to a first fraction (relatively smaller) of crushed material/mineral/ore to generate pellets/granules of a second fraction having a desired (relatively larger) size. Particles, pellets or granules of the granular substance are then selected by separating pellets/granules of a desired maximum and/or minimum size to form a fraction of material used to place within an enclosure.

In one further example, particles having a maximum circumferential size of 0.05 mm are selected to a third fraction of crushed material/mineral/ore. A binder is then added to the third fraction of crushed material/mineral/ore to generate pellets/granules of a fourth fraction having a desired (relatively larger) size. Particles, pellets or granules of the granular substance are then selected by separating or sifting pellets/granules of a desired maximum and/or minimum size to form a fraction of material used to place within an enclosure, e.g., a minimum size of 9 mm and a maximum size of 10 mm.

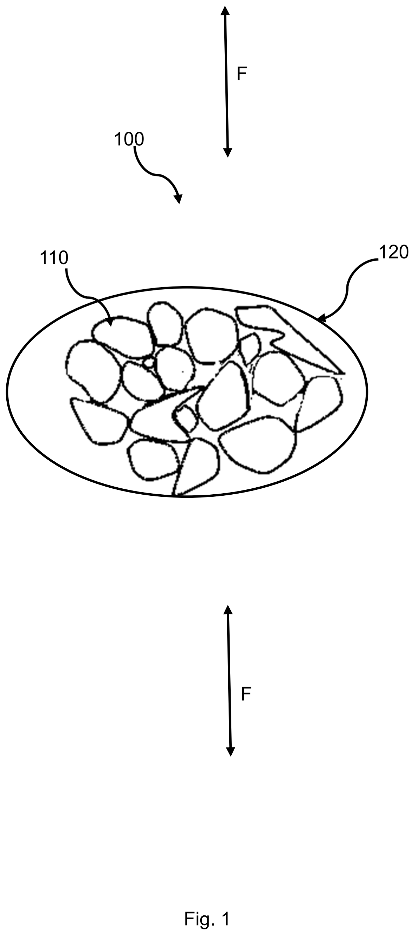

**Fig. 1** shows a bacteria and virus inhibiting arrangement 100 according to one or more embodiments of the present disclosure.

The straight arrows illustrate the direction of flow of one or more fluids F.

In one example, the arrangement is adapted for a floor drain and water is flowing in a direction from the top of the figure to the bottom of the figure whilst simultaneously sewage gas is flowing in a direction from the bottom of the figure to the top of the figure.

The arrangement 100 is configured to inhibit fungi, bacteria, virus and odors passing through the arrangement. In other words, the arrangement addresses not only symptoms like unpleasant odors but also the cause of such odors, which typically is bacteria. In particular, in clean environments such as a clean room, the arrangements inhibit or at least severely reduce the number of bacteria and virus entering a closed space, such as a clean room provided with a floor drain and/or an air vent.

Studies have been made in this respect, see e.g., "Vertical transmission of infectious aerosols through building toilet drainage system: An experimental study", https://doi.org/10.1016/j.envpol.2023.123284. This is e.g., relevant to transmission of SARS-Cov-2.

The arrangement comprises a granular substance 110 comprising crushed and calcinated mineral. Additionally, or alternatively, the granular substance is configured with a pH of the granular substance above 10.

In one embodiment, the granular substance comprises Calcium silicate. The Calcium silicate typically occurs naturally as mineral, and has a pH around 8-9. By subjecting the Calcium silicate to calcination, a process where the mineral is subjected to temperatures at or above 1000 degrees Celsius, the pH can be raised to greater than 10, or greater than 11 or greater than 12.

The inventors have realized that the relatively high pH reduces the amount of virus and/or bacteria in the fluid transmitted through the present arrangement.

In one embodiment, a fraction of, or the size of particles of, the granular substance is selected in proportion to a target flow of the fluid through the arrangement. In other words, when a relatively high inhibiting effect is desired and a relatively low flow is sufficient, a relatively small fraction may be used, e.g., 0.001-millimeter maximum particle size. When a relatively moderate inhibiting effect is desired and a relatively high flow is required, a relatively large fraction of the granular substance may be used, e.g., 10-millimeter maximum particle size.

In one example, in a floor drain where a relatively high flow of water down the drain is required, a fraction of 6 millimeters may be used. The exact fraction used can be selected using suitable methods using a desired target flow, e.g., as described in P.C. Carman, Fluid flow through granular beds, Chemical Engineering Research and Design, Volume 75, Supplement, 1997, Pages S32-S48, ISSN 0263-8762, https://doi.org/10.1016/S0263-8762(97)80003-2. (https://www.sciencedirect.com/science/article/pii/S0263876297800032).

In one embodiment, fractions of the granular substance are selected from a range of 0.001 millimeters to 10 millimeters of maximum particle size of the granular substance. In other words, granular substance particles are selected with a maximum size. Granular substance particles of the granular substance may be generated by crushing material/mineral/ore to a particular size and separating a desired fraction. Granular substance particles of the granular substance may further be generated by crushing material/mineral/ore to a relatively small size and forming relatively larger granules/pellets sizes by adding a binder to generate pellets/granules of a desired size. Particles of the granular substance are then selected by separating particles of a desired size to form a desired fraction of material used to place within an enclosure.

The arrangement further comprises a permeable enclosure 120 configured to hold the granular substance and to partially enclose fluid flowing through the granular substance to expose external surfaces of particles/granules/pellets of the granular substance to the fluid or vice versa.

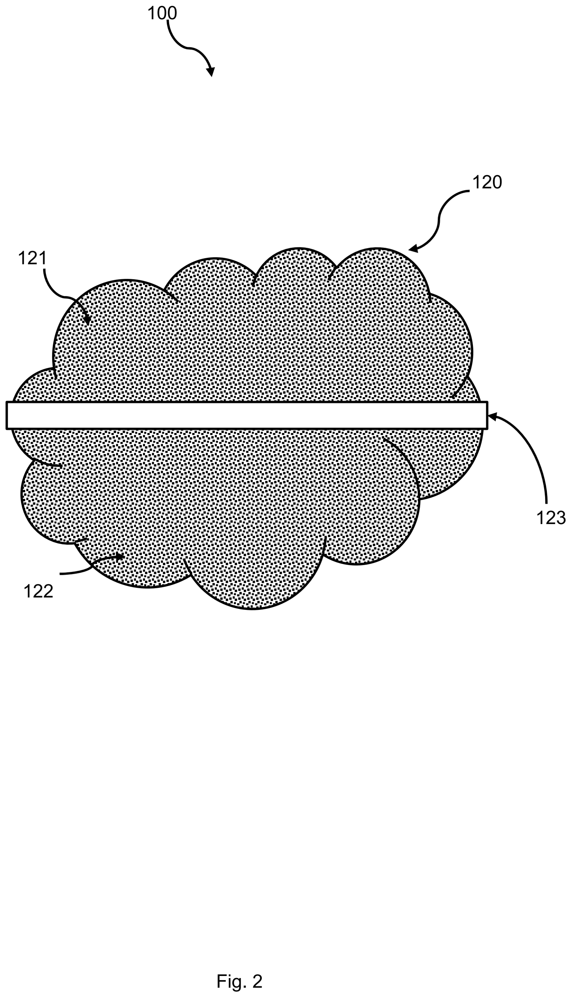

Details of enclosure 120 are further described in relation to Fig. 2.

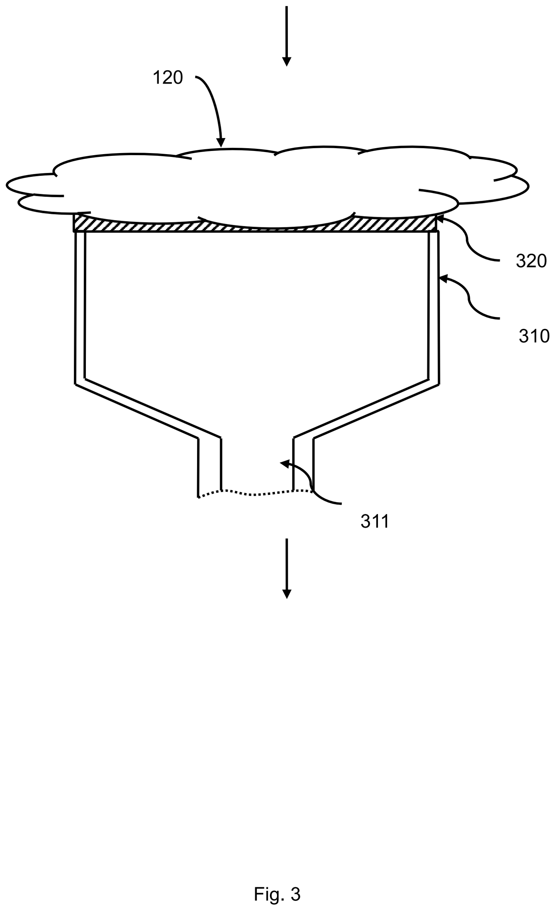

In one embodiment, the shape of the permeable enclosure is adapted to an outline of a floor drain to force fluid emanating from the floor drain to pass through the arrangement and be exposed to the granular substance. In one embodiment, the shape of the permeable enclosure is adapted to an outline of a cover or lid of the floor drain. In other words, the size of the enclosure is selected so that it completely covers a floor drain or an air vent. This is further described in relation to Fig. 3. Thereby, the fluid is forced to pass through the arrangement 100, and be exposed to the granular substance.

In one example, the permeable enclosure 120 comprises a top part of mesh material, and a lower part of mesh material that is welded together along a seam to hold the granular substance.

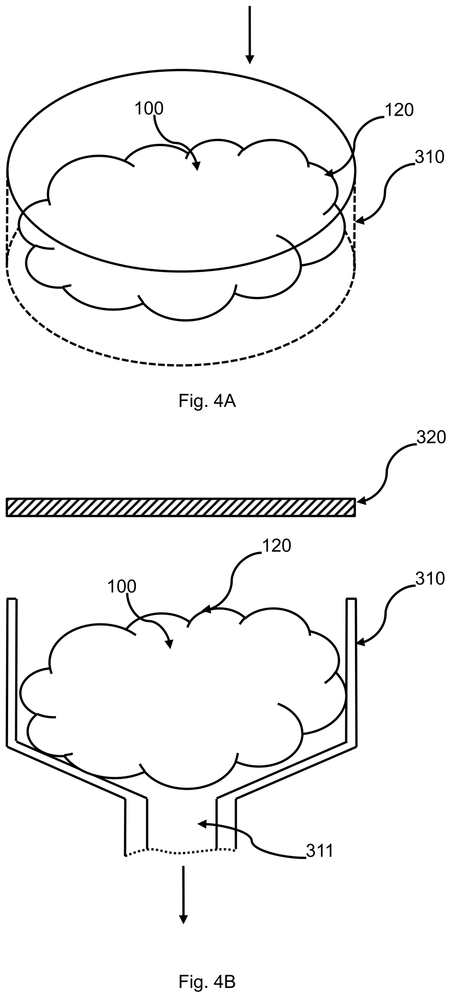

In one embodiment, the shape of the permeable enclosure is adapted to an interior of the floor drain. This is further described in relation to Fig. 4A and Fig. 4B. Thereby, the fluid is forced to pass through the arrangement 100, and be exposed to the granular substance.

In one embodiment, the shape of the permeable enclosure is adapted to a vent or air vent, to force fluid emanating from the vent to pass through the arrangement and be exposed to the granular substance. Additionally, or alternatively, the shape of the permeable enclosure is adapted to a filter to force fluid received from an inlet to pass through the arrangement and be exposed to the granular substance. Thereby, the fluid is forced to pass through the arrangement 100, and be exposed to the granular substance.

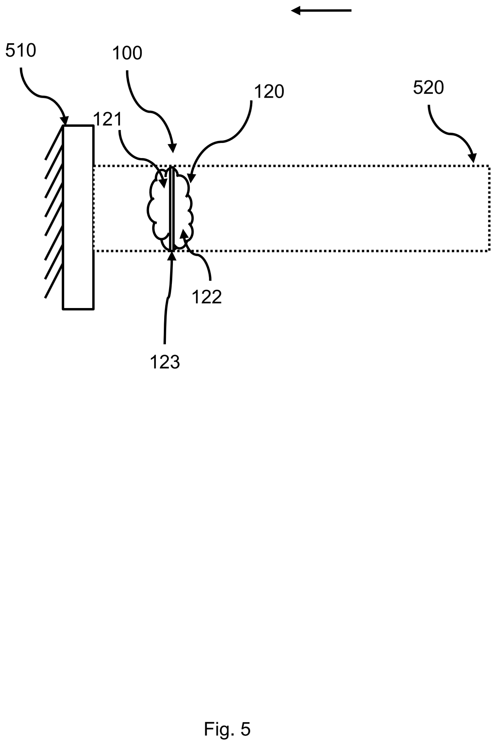

This is further described in relation to Fig. 5.

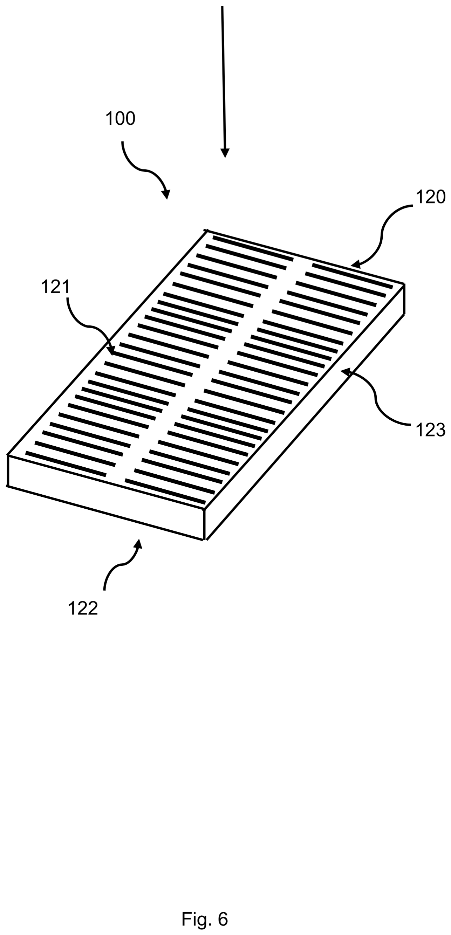

In one embodiment, the shape of the permeable enclosure is adapted to a face mask, to force fluid inhaled by a user of the face mask to pass through the arrangement and be exposed to the granular substance. This is further described in relation to Fig. 6. Thereby, the fluid is forced to pass through the arrangement 100, and be exposed to the granular substance.

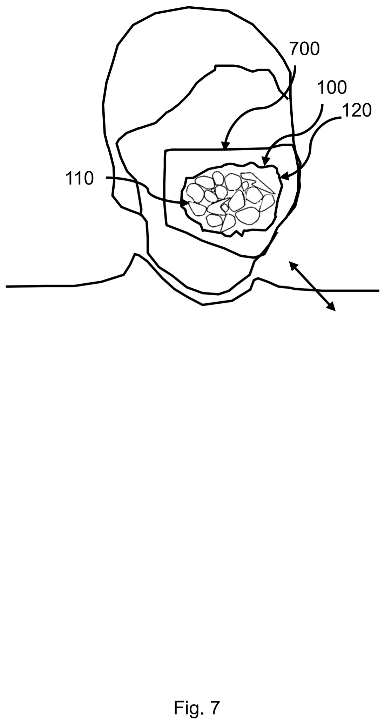

Additionally, or alternatively, the shape of the permeable enclosure is adapted to an air-filter of a vehicle. This is further described in relation to Fig. 7.

**Fig. 2** shows details of the arrangement 100 according to one or more embodiments of the present disclosure.

In this embodiment, the permeable enclosure 120 comprises a first permeable part 121, a second permeable part 122 and a third non-permeable part 123, wherein the third non-permeable part (123) is joined or coupled to the first permeable part (121) and to the second permeable part (122) to hold the granular substance.

In one example, the first permeable part 121 and the second permeable part 122 comprises a mesh material, e.g., a plastic net. The first permeable part 121 and the second permeable part 122 may be welded together to form the third non-permeable part 123, i.e., a seam where the first and second parts are joined. By welding the first permeable part 121 and the second permeable part 122 together, the granular substance is maintained within the arrangement 100.

The first permeable part 121 and the second permeable part 122 may comprise any suitable material that will hold the granular substance and simultaneously allow fluid to pass through the arrangement 100, without departing from the present disclosure. Further examples may include metal or degradable and/or environmentally friendly materials.

In one embodiment, the third non-permeable part 123 may further be configured to form a seal to a surrounding fluid conduit or guide, e.g., to form a seal to the interior of a floor drain, the interior of an air duct or the interior of a filter holder.

In one example, the third non-permeable part 123 comprises silicone or soft plastic material shaped to align with a shape of the fluid conduit or guide. The third non-permeable part 123 may e.g., be shaped to the interior of an air duct, effectively forming an air-tight seal that forces air travelling through the air duct to pass through the arrangement 100 and thereby be exposed to the granular substance 110.

**Fig. 3** illustrates an example where the shape of the permeable enclosure is adapted to an outline of a floor drain according to the present disclosure.

The straight arrows indicate the direction of flow of water through the floor drain.

The floor drain 310 is configured with an outlet 311, which is fluidly coupled to the sewage system, typically via conduits or pipes. The floor drain 310 is further configured with a cover or lid 320 having a predetermined size or area.

In embodiments, the shape of the permeable enclosure may be selected to exceed the predetermined size or area of the floor drain 310, thereby forcing fluid or gas emanating from the floor drain to pass through the arrangement and be exposed to the granular substance. This is typically sewage gas having an unpleasant odor, fungi, bacteria or virus.

Bacteria, virus and smell of sewage gas is then inhibited, eliminated or greatly reduced when passing through the arrangement 100.

**Fig. 4A** illustrates a perspective view of an embodiment where the shape of the permeable enclosure is adapted to an interior of a floor drain according to the present disclosure.

The straight arrow indicates the direction of flow of water through the floor drain.

The floor drain 310 is configured with an outlet (not shown), which is fluidly coupled to the sewage system, typically via conduits or pipes. The floor drain 310 is further configured with an interior having a predetermined size, volume or area.

In embodiments, the shape of the permeable enclosure 120 may be selected to match the predetermined size, volume or area of the interior of the floor drain 310, thereby forcing fluid , e.g., gas, emanating from the sewage to pass through the arrangement and be exposed to the granular substance. This is desirable, as typically sewage gas has an unpleasant odor and bacteria and/or virus may contaminate samples in a clean room.

Bacteria, virus and smell of sewage gas is then eliminated or greatly reduced when passing through the arrangement 100.

**Fig. 4B** illustrates a side view of an embodiment where the shape of the permeable enclosure is adapted to an interior of a floor drain according to the present disclosure.

The straight arrow indicates the direction of flow of water through the floor drain.

The floor drain 310 is configured with an outlet 311, which is fluidly coupled to the sewage system, typically via conduits or pipes. The floor drain 310 is further configured with an interior having a predetermined size or area.

As shown in Fig. 4A, the shape of the permeable enclosure 120 may be selected to match the predetermined size or area of the interior of the floor drain 310, thereby forcing fluid or gas emanating from the sewage to pass through the arrangement and be exposed to the granular substance. This is typically sewage gas having an unpleasant odor.

Bacteria, virus and smell of sewage gas is then eliminated or greatly reduced when passing through the arrangement 100.

**Fig. 5** illustrates a side view of an embodiment where the shape of the permeable enclosure is adapted to a vent or a filter according to the present disclosure.

The straight arrow indicates the direction of flow of air through an air duct or conduit 520.

As can be seen in Fig. 5, the air duct 520 provides a flow of air to a vent/air vent 510. Fins provided on the air vent directs the ai downwards.

In one embodiment, the shape of the permeable enclosure 120 is adapted to the vent 510. In other words, the shape of the permeable enclosure 120 is adapted to form a seal to the vent 510 such that air received from the air duct 520 is forced to flow through the arrangement 100. In one example, the third non-permeable part 123 may be configured to act as a seal between the arrangement 100 and the air duct 520.

With reference to Fig. 2, the arrangement may comprise a first part of permeable material 121, a second part of permeable material 122 and a third part of non-permeable material. In one example, the first and second parts are made from paper and the third part is made from silicone that seals to a frame of the vent 510. In other words, the third part forms an airtight seal to the vent 510.

In one embodiment, the shape of the permeable enclosure 120 is adapted to a filter and/or a conduit, such as the air duct 520. In other words, the shape of the permeable enclosure 120 is adapted to form a seal to a filter holder or to the air duct 520 to force air received travelling through the air duct 520 to flow through the arrangement 100.

With reference to Fig. 2, the arrangement may comprise a first part of permeable material 121, a second part of permeable material 122 and a third part of non-permeable material. In one example, the first and second parts are made from paper and the third part is made from silicone, or any other suitable material, that seals to a filter holder or the air duct 520. In other words, the third part forms an airtight seal to the air duct 520.

**Fig. 6** illustrates a perspective view of an embodiment where the shape of the permeable enclosure is adapted to an air filter of a vehicle according to the present disclosure.

The straight arrow indicates the direction of flow of air through the air filter 100.

In this embodiment, the shape of the permeable enclosure 120 is adapted to an air filter of a vehicle. In other words, the shape of the permeable enclosure 120 is adapted to form a seal 123 to a filter holder (not shown) to force air received travelling through an air duct (not shown) to flow through the arrangement 100.

With reference to Fig. 2, the arrangement 100 may comprise a first part of permeable material 121, a second part of permeable material 122 and a third part of non-permeable material 123. In one example, the first and second parts are made from paper and the third part 123 is made from silicone that seals to a filter holder of the air filter. In other words, the third part forms an airtight seal to the filter holder.

**Fig. 7** illustrates a perspective view of an embodiment where the shape of the permeable enclosure is adapted to a face mask 700 according to the present disclosure.

This forces fluid inhaled or exhaled by a user of the face mask 700 to pass through the arrangement 100 and be exposed to the granular substance. Thereby the flow of bacteria, virus and/or odor flowing through the arrangement 100 is inhibited or at least greatly reduced.

The straight arrow indicates the direction of flow of air through the face mask 100.

In this embodiment, the shape of the permeable enclosure 120 is adapted to face mask. In other words, the shape of the permeable enclosure 120 is adapted to form a seal to a filter holder (not shown) or the face mask such that air received travelling through the face mask is forced to flow through the arrangement 100.

Additionally, or alternatively, the arrangement 100 may be configured as a replaceable filter unit, or be integrated into the face mask to form an integral part of the face mask 700.

With reference to Fig. 2, the arrangement 100 may comprise a first part of permeable material 121, a second part of permeable material 122 and a third part of non-permeable material 123. In one example, the first and second parts are made from paper and the third part is made from silicone that seals to a filter holder and/or air channels of the face mask 700. Any suitable filter material or sealing material may be used without departing from the present disclosure. In other words, the third part forms an airtight seal to the face mask 700, directly or via the filter holder.

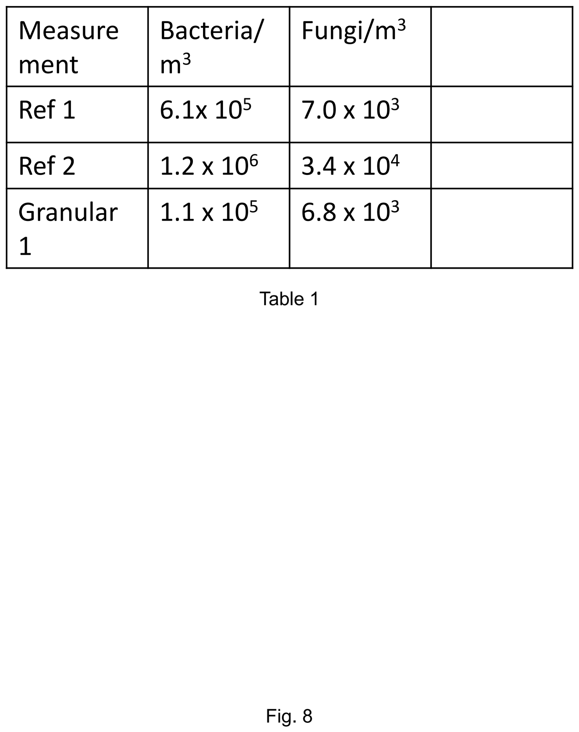

**Fig. 8** illustrates a comparison between not using and using the arrangement 100 according to the present disclosure.

To compare the effect of the arrangement, tests were performed at Lindholmens Sewage Treatment Plant, Norrtälje, Sweden May 23 2023. Tests were conducted on air at a sewage inlet pump by a certified laboratory. The tests revealed a total number of bacteria and fungi per cubic meter.

Two reference measurements, Ref 1 and Ref 2 were performed, without the arrangement of the present disclosure. A further measurement where the arrangement of the present disclosure was used was made.

As can be seen in Table 1, the levels of bacteria and fungi were significantly reduced.

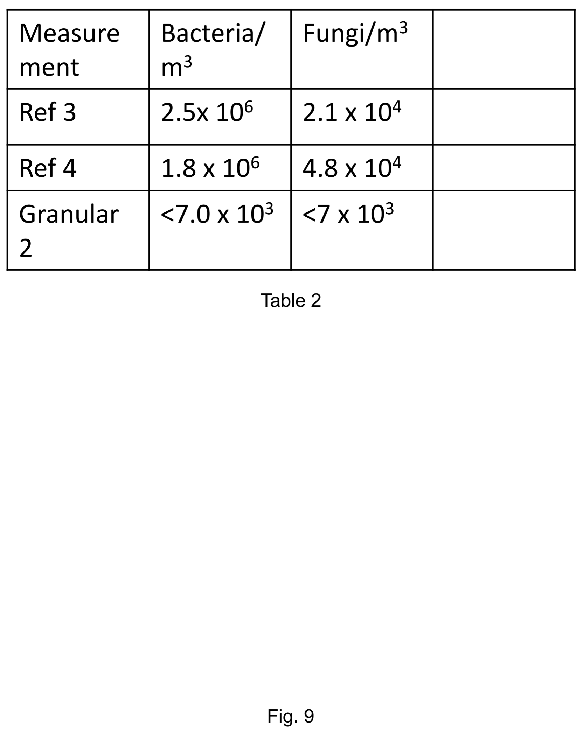

**Fig. 9** illustrates a second comparison between not using and using the arrangement 100 according to the present disclosure.

To compare the effect of the arrangement, tests were performed at Lindholmens Sewage Treatment Plant, Norrtälje, Sweden June 20, 2023. Tests were conducted on air at a sewage inlet pump by a certified laboratory. The tests revealed a total number of bacteria and fungi per cubic meter.

Two reference measurements, Ref 3 and Ref 4 were performed, without the arrangement of the present disclosure. A further measurement where the arrangement of the present disclosure was used was made.

As can be seen in Table 2, the levels of bacteria and fungi were significantly reduced.

Finally, it should be understood that the invention is not limited to the embodiments described above, but also relates to and incorporates all embodiments within the scope of the appended independent claims.

## Claims

1. An bacteria and virus inhibiting arrangement (100), the arrangement comprising:
a granular substance (110) comprising crushed and calcinated mineral, wherein a pH of the granular substance is above 10,
a permeable enclosure (120) configured to hold the granular substance and to partially enclose fluid flowing through the granular substance to expose external surfaces of the granular substance to the fluid.

2. The arrangement according to claim 1, wherein the granular substance comprises Calcium silicate.

3. The arrangement according to claims 1 or 2, wherein fractions of the granular substance are selected in proportion to a target flow of the fluid through the arrangement.

4. The arrangement according to claim 3 ,wherein fractions of the granular substance are selected within a range of 0.001 millimeters to 10 millimeters.

5. The arrangement according to any of the preceding claims, wherein the shape of the permeable enclosure is adapted to an outline of a floor drain to force fluid emanating from the floor drain to pass through the arrangement and be exposed to the granular substance.

6. The arrangement according to claim 4, wherein the shape of the permeable enclosure is adapted to an interior of the floor drain.

7. The arrangement according to claim 4, wherein the shape of the permeable enclosure is adapted to an outline of a cover of the floor drain.

8. The arrangement according to any of the preceding claims, wherein the shape of the permeable enclosure is adapted to a vent, to force fluid emanating from the vent to pass through the arrangement and be exposed to the granular substance.

9. The arrangement according to any of the preceding claims, wherein the shape of the permeable enclosure is adapted to a face mask, to force fluid inhaled by a user of the face mask to pass through the arrangement and be exposed to the granular substance.

10. The arrangement according to any of the preceding claims, wherein the shape of the permeable enclosure is adapted to a filter to force fluid received from an inlet to pass through the arrangement and be exposed to the granular substance.

11. The arrangement according to claim 10, wherein the shape of the permeable enclosure is adapted to an air-filter of a vehicle.

12. The arrangement according to any of the preceding claims, wherein permeable enclosure (120) comprises a first permeable part (121), a second permeable part (122) and a third non-permeable part (123), wherein the third non-permeable part (123) is joined to the first permeable part (121) and to the second permeable part (122) to hold the granular substance.
